# EUROPÄISCHE PATENTSCHRIFT

(11) **EP 1 589 094 B1**
(45) Veröffentlichungstag und Bekanntmachung des Hinweises auf die Patenterteilung: **16.04.2008**
(21) Anmeldenummer: 05008575.2
(22) Anmeldetag: 20.04.2005
(51) Int. Cl.: C12M 1/10, C12M 1/107

(54) **Drehbarer Fermenter**
Rotatory fermenter
Fermenteur rotatif

(30) Priorität: 23.04.2004 DE 202004006610 U
(43) Veröffentlichungstag der Anmeldung: 26.10.2005
(73) Patentinhaber: Infors AG, 4103 Bottmingen (CH)
(72) Erfinder: Hawrylenko, Alexander, 4103 Bottmingen (CH)
(74) Vertreter: Säger, Manfred

(56) Entgegenhaltungen:
- EP-A- 0 495 315
- DE-U1- 29 903 405
- US-A- 5 403 742

## Beschreibung

Die vorliegende Erfindung betrifft einen Fermenter gemäß dem Oberbegriff des Hauptanspruchs, nämlich einen Fermenter mit einen an einem Auflager angeordneten gasdichten, drehbaren Behälter, mit einer Beschickungs- bzw. Entnahmeöffnung zum Innenraum des Behälters, mit einem zu dessen Rotation dienenden Drehantrieb, mit einer an dem Auflager festgelegten feststehenden Achse, über deren Innenkanäle Gas und/oder Flüssigkeiten zu- sowie abführbar ist, mit einem in Bezug auf die Rotation im Bereich des Pols des Behälters starr angebrachten Behälterkopf, und mit einem einerseits an diesem festgelegten und andererseits mit einem die Achse umgreifenden und sich an dieser abstützenden Drehlager versehen ist, wobei die Achse im Betrieb des Fermenters horizontal angeordnet ist.

Solche Fermenter sind bekannt (DE-U 299 03 405) und enthalten einen Behälter für das biochemisch im Rahmen einer Reaktion zu behandelnde Gut, sowie ggf. ein Rührelement, das für die notwendige Durchmischung des Reaktionsgutes sorgt.

Dieser bekannte Fermenter weist an einander gegenüberliegenden Polen angeordnete Drehlager auf, von denen eines zugleich mit dem Drehantrieb versehen ist. In dem Äquatorialbereich zwischen den Polen sind die Beschickungs- und Entnahmeöffnung für das Reaktionsgut angeordnet.

Gegenüber üblichen chemischen Reaktoren ist es bei Fermentern zur Erzielung eines guten Wirkungsgrades erforderlich, die Temperatur des Reaktionsgutes zu beeinflussen. Dies ist bei dem bekannten Fermenter nicht vorgesehen und allenfalls dadurch möglich, dass seine gesamte Umgebung geheizt wird, wobei auch an eine Kapselung des gesamten bekannten Fermenters gedacht werden könnte.

Der Erfindung liegt deshalb die Aufgabe zugrunde, einen gattungsgemässsen Fermenter gemäss dem Oberbegriff des Hauptanspruchs so weiterzubilden, dass damit auch eine temperaturabhängige Behandlung von Reaktionsgütern möglich ist.

Diese Aufgabe wird bei einem gattungsgemässsen Fermenter gemäss dem Oberbegriff des Hauptanspruchs erfindungsgemäß durch dessen kennzeichnende Merkmale gelöst.

Mit dem erfindungsgemässen Fermenter ist ein völlig anderes Prinzip als beim gattungsgemässen Stand der Technik verwirklicht. Nachdem beim Betrieb des Fermenters die Achse horizontal verläuft, kommt das Reaktionsgut im äquatorialen Bereich zwischen den beiden Polen des Innenraums mit der Behälterwandung zur Anlage, der erfindungsgemäss doppelwandig unter Bildung eines Hohlraums ausgebildet ist und der zum Durchlauf eines Fluids, vorzugsweise Wasser mit einer vorgebbaren Temperatur nutzbar ist. Wegen der Möglichkeit der Steuerung der Temperatur lässt der erfindungsgemäße Fermenter eine bessere Effizienz sowie Steuerung der technischen, biochemischen und mikrobiologischen Parameter des Reaktionsgutes zu. Desweiteren ist der Behälter des Fermenters nur mit einem einzigen polseitigen Drehlager versehen, wodurch der mit Bezug auf den äquatorseitigen Hohlraums andere Pol konstruktionsbedingt unbenutzt ist, weshalb dort mit Vorteil die mit einem Deckel verschliessbare Beschickungs- bzw. Entnahmeöffnung angeordnet ist. Um bei der Erfindung die Beschickung und die Entnahme zu erleichtern, ist zwischen dem Auflager und der Achse ein Kipplager mit einer Kippachse vorgesehen ist, die vorzugsweise rechtwinklig zu der Achse und horizonal angeordnet ist.

Es ist zwar ein hohlzylindrischer Fermenter mit einer zwecks Temperierung doppelwandig ausgeführten Mantels bekannt (EP-A-0495315); dessen beide Stirnseiten sind aber nicht dicht verschlossen, so dass dort Gas und zu behandelndes Gut austreten kann.

Ein besonders einfacher Drehantrieb ergibt sich, wenn der Behälterkopf des Behälters einen zylinderförmigen Mantel aufweist und an diesem im Betrieb des Fermenters zumindest ein Treibglied des Drehantriebs reibungsschlüssig angreift.

Weitere zweckmässige Ausgestaltungen und Weiterbildungen der Erfindung sind in den Unteransprüchen gekennzeichnet.

Die Erfindung wird nachfolgend anhand der Zeichnung näher erläutert, die den erfindungsgemässen Fermenter, im schematischen Längsschnitt zeigt.

Der Fermenter 5 ist auf einem insgesamt mit 6 bezeichneten Auflager angeordnet und weist einen gasdichten drehbaren, mit einem Innenraum 71 versehenen Behälter 7 für das zu bearbeitende Reaktionsgut auf. An dem Auflager 6 ist an dessen oberem Ende eine Kippachse 81 aufweisendes Kipplager 8 vorgesehen, an welchem eine insgesamt mit 9 bezeichnete Achse starr festgelegt ist. Über Innenkanäle 91, 92 kann dem Innenraum 71 des Behälters Gas und/oder Flüssigkeiten zugeführt beziehungsweise abgeführt werden.

Der Behälter 7 ist ferner in Bezug auf die Drehbewegung bei seiner Rotation um die feststehende Achse 9 im Bereich des einen Pols des Behälters mit einer starr an diesem festgelegten Behälterkopf 72 versehen. Dieser Behälterkopf 72 weist zwei die Achse 9 umgreifende, sich an dieser abstützende Drehlager 73, 74 auf, sodass sich der Behälter 7 mittels der Drehlager 73, 74 um die feststehende Achse 9 drehen kann, wobei diese im Betrieb des Fermenters horizontal angeordnet ist.

Der Behälter 7 ist an seinem sich an den den Behälterkopf 72 aufweisenden Polbereich anschliessenden und mit Bezug auf seine Rotation äquatorialen Bereich doppelwandig unter Bildung eines Hohlraums 75 ausgebildet, der mit einer Zu- und Ablaufleitung 76, 77 versehen ist. Diese Zubeziehungsweise Ablaufleitung 76, 77 sind in dem Behälterkopf 72 vorgesehene Ringräume 78 beziehungsweise 79 geführt. In diese Ringräume 78, 79 münden im Innern der Achse 9 geführte Zubeziehungsweise Ablaufkanäle 10, 11. Diese sind bis hinter das Kipplager 8 geführt und weisen dort -nicht gezeigte- gezeigte Anschlüsse für ein Wärmeträgermedium, beispielsweise Wasser auf.

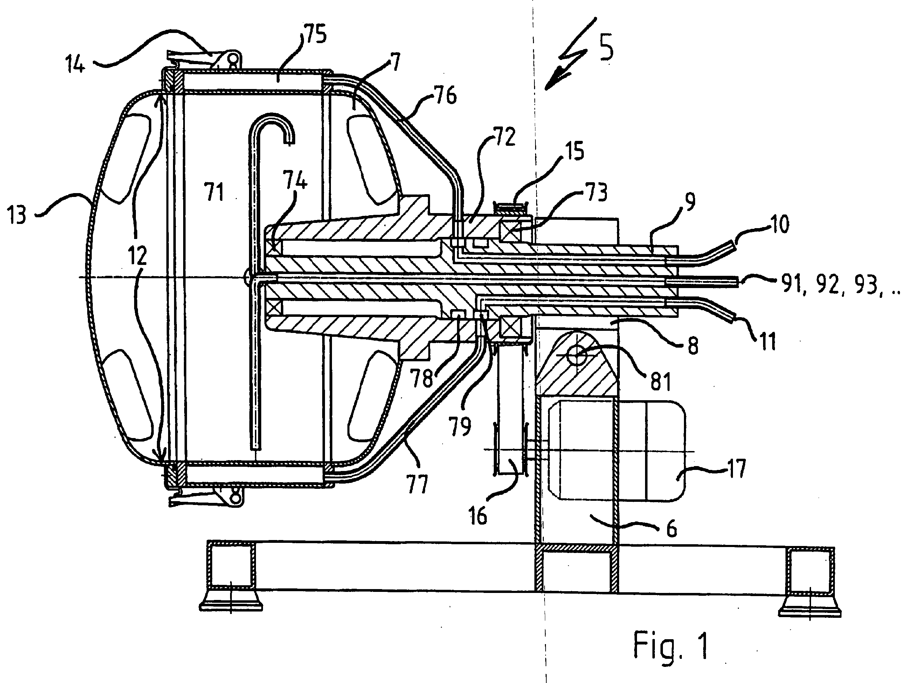

Wegen des nur an einem der beiden Pole vorgesehenen Drehlagers 73, 74 ist an dem auf der anderen Seite des Äquators des Behälters 7 liegenden Bereich eine insgesamt mit 12 bezeichnete Beschickungsbeziehungsweise Entnahmeöffnung vorgesehen, die mittels eines Deckels 13 verschliessbar ist. Dieser Deckel 13 ist mittels einer Arretiervorrichtung 14 im äquatorialen Bereich des Behälters 7 lösbar festgelegt. In Verbindung mit dem Kipplager 8 kann also der Fermenter 5 von der in Figur 1 gezeigten -in Figur 1 im Uhrzeigersinn- in eine nahezu vertikale Lage gebracht werden, sodass der Behälter in dieser Lage beschickt und/oder das Reaktionsgut nach Öffnen des Deckels 13 entnommen werden.

Hierzu ist die Kippachse 81 des Kipplagers 8 rechtwinklig zu der Achse 9 sowie mit Bezug auf das Auflager 6 horizontal angeordnet.

Ferner weist der Behälterkopf 72 einen zylinderförmigen Mantel 15 auf, an dem im Betrieb des Fermenters 5 zumindest ein Treibglied 16 des Drehantriebs 17 reibungsschlüssig angreift.

## Patentansprüche

1. Fermenter mit einen an einem Auflager (6) angeordneten gasdichten, drehbaren Behälter (7), mit einer Beschickungs- bzw. Entnahmeöffnung (12) zum Innenraum (71) des Behälters (7), mit einem zu dessen Rotation dienenden Drehantrieb (17), mit einer an dem Auflager festgelegten feststehenden Achse (9), über deren Innenkanäle (91,92) Gas und/oder Flüssigkeiten zu- sowie abführbar ist, mit einem in Bezug auf die Rotation im Bereich des Pols des Behälters starr angebrachten Behälterkopf (72), und mit einem einerseits an diesem festgelegten und andererseits mit zumindest einem die Achse (9) umgreifenden und sich an dieser abstützenden Drehlager (73,74) versehen ist, wobei die Achse im Betrieb des Fermenters (5) horizontal angeordnet ist, **dadurch gekennzeichnet, dass** der Behälter (7) -mit Bezug auf seine Rotation- im Bereich seines Äquators unter Bildung eines Hohlraums (75) doppelwandig ausgebildet ist, dass dieser Hohlraum (75) mit einer Zu- sowie einer Ablaufleitung (76,77) versehen ist, dass diese Zu- sowie Ablaufleitung zu abgedichteten Ringräumen (78,79) in dem Behälterkopf (72) geführt sind, in die im Inneren der Achse geführte Zu- bzw. Ablaufkanäle münden, dass der Behälter (7) des Fermenters (5) mit an nur einem der beiden Pole vorgesehenen einzigen polseitigen Drehlager (73,74) versehen ist, dass zwischen dem Auflager (6) und der Achse (9) ein Kipplager (8) mit einer Kippachse (81) vorgesehen ist, die vorzugsweise rechtwinklig zu der Achse (9) und horizonal angeordnet ist und dass der auf der bezüglich des einen Pols anderen Seite des Äquators des Behälters (7) liegenden Bereich mit einer Beschickungsbeziehungsweise Entnahmeöffnung (12) versehen ist, die mittels eines Deckels 13 verschliessbar ist.

2. Fermenter nach Anspruch 1, **dadurch gekennzeichnet, dass** der Behälter (6) in seinem äquatorialen Bereich zumindest teilweise eine zylinderförmig umlaufende Wandung (15) aufweist und an dieser im Betrieb des Fermenters (5) zumindest ein Treibrad (16) des Drehantriebs (17), deren Achsen (18) parallel zu der Achse (9) angeordnet ist, reibungsschlüssig angreift.

3. Fermenter nach Anspruch 1 , **dadurch gekennzeichnet, dass** der Behälterkopf (72) des Behälters (6) einen zylinderförmigen Mantel (15) aufweist und an diesem im Betrieb des Fermenters (5) zumindest ein Treibglied (16) des Drehantriebs (17) reibungsschlüssig angreift.

## Claims

1. Fermenter with a gas-tight, rotatable container (7) disposed on a support (6),
with an opening (12) for charging and discharging the interior chamber (71) of the container (7),
with a rotary drive (17) for the purpose of driving the latter,
with a fixed axle (9) rigidly mounted on the support providing internal channels (91, 92), through which gas and/or liquids can be supplied and drained,
with a container head (72) mounted in a rotationally-rigid manner in the region of the pole of the container,
and with on the one hand at least one rotary bearing (73,74) rigidly attached to the latter, and, on the other hand, engaging around and supported against the axle (9), wherein the axle is arranged horizontally during the operation of the fermenter (5),
**characterised in that**
with reference to its rotation, the container (7) is designed with a double wall thereby forming a hollow cavity (75) in the region of its equator,
that this hollow cavity (75) is provided with supply and drainage lines (76, 77),
that these supply and drainage lines are guided to sealed annular chambers (78, 79) within the container head (72), into which supply and drainage channels guided through the interior of the axle open;
that the container (7) of the fermenter (5) is fitted with a single, rotary bearing (73, 74) at the pole-end provided only at one of the two poles;
that a tilting bearing (8) with a tilting axle (81) is provided between the support (6) and the axle (9), which is preferably disposed horizontally and at right angles to the axle (9);
and that the region disposed on the other side of the equator of the container (7) relative to the one pole is provided with an opening (12) for charging and respectively discharging, which can be closed by means of a cover (13).

2. Fermenter according to claim 1,
**characterised in that**
the container (7) provides a wall (15) extending at least partially in a cylindrical manner in its equatorial region, which engages in a frictional manner during the operation of the fermenter (5) with at least one drive wheel (16) of the rotary drive (17), of which the axle (18) is arranged parallel to the axle (9).

3. Fermenter according to claim 1,
**characterised in that**
the container head (72) of the container (7) provides a cylindrical casing (15), which engages in a frictional manner during the operation of the fermenter (5) with at least one drive element (16) of the rotary drive (17).

## Revendications

1. Fermenteur comportant un récipient (7) rotatif et étanche aux gaz, disposé sur un support (6), comportant une ouverture de chargement ou de prélèvement (12) menant vers l'espace interne (71) du récipient (7), un entraînement rotatif (17) servant à sa rotation, un axe (9) immobile, fixé sur le support, par l'intermédiaire des canaux internes (91, 92) duquel du gaz et/ou des liquides peuvent être délivrés ou évacués, comportant également une tête de récipient (72) appliquée de manière stable dans la zone du pôle du récipient, par rapport à la rotation, et au moins un coussinet de pivotement (73, 74) qui, d'une part, est fixé sur celle-ci et, d'autre part, enveloppe l'axe (9) et repose en outre sur celui-ci, moyennant quoi l'axe est disposé horizontalement pendant le fonctionnement du fermenteur (5), **caractérisé en ce que** le récipient (7) - par rapport à sa rotation - est configuré avec une double paroi en formant un espace creux (75) dans la zone de son équateur, **en ce que** cet espace creux (75) est doté d'une conduite d'alimentation ainsi que d'une conduite d'évacuation (76, 77), **en ce que** ces conduites d'alimentation et d'évacuation sont guidées vers des espaces annulaires étanchéifiés (78, 79) dans la tête du récipient (72), dans lesquels débouchent les canaux d'alimentation ou d'évacuation guidés à l'intérieur de l'axe, **en ce que** le récipient (7) du fermenteur (5) est doté d'un coussinet de pivotement (73, 74) unique du côté pôle, prévu sur seulement l'un des deux pôles, **en ce que**, entre le support (6) et l'axe (9), est prévu un appui oscillant (8) avec un axe d'oscillation (81), lequel est disposé de préférence perpendiculairement à l'axe (9) et horizontalement, et **en ce que** la zone se trouvant de l'autre côté de l'équateur du récipient (7), par rapport à l'un des pôles, est dotée d'une ouverture de chargement ou de prélèvement (12), qui peut être fermée à l'aide d'un couvercle (13).

2. Fermenteur selon la revendication 1, **caractérisé en ce que** le récipient (6) présente, dans sa zone équatoriale, au moins en partie une paroi (15) périphérique de forme cylindrique et, lors du fonctionnement du fermenteur (5), au moins une roue motrice (16) de l'entraînement rotatif (17) s'engage avec celle-ci par friction, l'axe (18) de la roue motrice étant disposé parallèlement à l'axe (9).

3. Fermenteur selon la revendication 1, **caractérisé en ce que** la tête (72) du récipient (6) présente une enveloppe (15) de forme cylindrique, et, pendant le fonctionnement du fermenteur (5), au moins un organe moteur (16) de l'entraînement rotatif (17) s'engage par friction avec celle-ci.
